# EUROPEAN PATENT APPLICATION

(11) **EP 3 335 717 A1**
(43) Date of publication of application: **20.06.2018**
(21) Application number: 16203846.7
(22) Date of filing: 13.12.2016
(51) Int. Cl.: A61K 36/88, A61K 36/899, A61K 36/15, A23L 33/105, A61P 15/00, A61P 25/24

(54) **COMPOSITION FOR USE IN TREATING SYMPTOMS RELATING TO PREMENSTRUAL SYNDROME (PMS)**

(71) Applicant: Serelys Pharma S.A.M., 98000 Monaco (MC)
(72) Inventor: MORRA, Sossio, 98000 Monaco (MC)
(74) Representative: Axe PI

(57) **Abstract**

The current invention concerns a composition for use in treating symptoms relating to premenstrual syndrome (PMS) characterized in that said composition comprises at least one pollen or pistil extract or a combination of the latter as first active ingredient and at least one second active ingredient, said second active ingredient is herbal or botanical material derived component and suitable of treating depressive and/or dysphoria symptoms linked to PMS.

## Description

### TECHNICAL FIELD

The invention pertains to the technical field of formulations for use in treating symptoms relating to PMS.

### BACKGROUND

The term premenstrual syndrome, or PMS, is generally used to describe a group of physical and mental symptoms which occur cyclically beginning about seven to fourteen days prior to menses. Menstruation occurs in women from the age of about twelve to thirteen to, on average, until about forty seven years of age. It occurs at more or less regular intervals except during pregnancy and lactation. The normal menstrual cycle averages about twenty-eight days with some variation based upon the woman's genetic makeup, age, physical and emotional well-being, as well as other factors. The duration of menstrual flow is variable but usually is between three and seven days. The symptoms of PMS are often so severe and widespread that the American Psychiatric Association has formally identified the diagnostic criteria for PMS in Diagnostic and Statistical Manual of Mental Disorders.

The specific etiology of PMS remains unknown, although many theories have been proposed. These theories include, but are not limited to: hormonal imbalances, hormonal deficiencies, vitamin deficiencies, disturbances of autonomic nervous system, salt and water imbalances, altered endogenous opiates such as endorphins and psychosomatic dysfunction, just to name a few. However, up to now, investigative studies of etiology have been inconclusive and sometimes conflicting. Most likely, PMS is multifactorial and probably also involves changes in neurohormones and neurotransmitters, which are difficult to observe, document and isolate in vivo.

Symptoms of PMS are varied and can range from mild to incapacitating. As many as seventy to ninety percent of all women have recurrent premenstrual syndrome, and as many as twenty to forty percent of these women suffer some degree of temporary physical and/or mental incapacitation. Some examples of mental symptoms a woman suffering from PMS may exhibit include difficulty in concentration, fatigue, change in appetite, irritability and depression. Some examples of physical symptoms a woman suffering from PMS may exhibit are increase or decrease in sleep, joint pain, cramps, bloating, edema, acne, constipation and breast tenderness.

Compositions comprising extracts of pollen and/or pistils for combatting symptoms relating to PMS have been previously described in the art. As these compositions are all natural, they provide an attractive treatment for women suffering from PMS.

However, recent hitherto unknown studies have shown that although these compositions are indeed helpful in treating or alleviating a large part of symptoms relating to PMS, these compositions show less or no efficacy towards symptoms relating to depression and dysphoria. In fact, it was surprisingly reported in this novel study that in women for whom the most severe PMS symptom was depression and/or dysphoria, these pollen extract based compositions have almost no effect.

Hence, there is a need in the art for compositions which are able to combat all symptoms of PMS, including those that concern mood swings, forms of depression and general dysphoria. By preference, such composition is of natural origin.

The invention thereto aims to provide a composition which is suitable for combatting a broad range of a symptoms relating to PMS, including those directed to depression and dysphoria. The composition is an all-natural composition, and can be taken without any risk for unwanted side-effects.

### SUMMARY OF THE INVENTION

The present invention provides a composition according to claim 1, which provides an optimal, plant based treatment for treating and/or preventing a full range of symptoms relating to PMS, including dysphoria and depression. The composition is hence suitable for all patients reporting one or more PMS symptoms, and is not restricted to those reporting classical symptoms. As a consequence, the composition according to the current invention is broadly applicable and highly efficient.

### FIGURES

Figure 1 shows the differences observed in a patient group suffering from classic PMS symptoms and those suffering from depression and/or dysphoria when treated with a composition based on a pollen/pistil extract.
Figure 2 shows the differences in weight gain prior to the menses in a patient group suffering from classic PMS symptoms and those suffering from depression and/or dysphoria.

### DETAILED DESCRIPTION OF THE INVENTION

The present invention concerns a novel composition for use in treating symptoms relating to premenstrual syndrome (PMS). The composition provides an effective solution for all symptoms relating to PMS, including depression, dysphoria and/or mood swings.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise," "comprising," and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints.

The expression "% by weight" (weight percent), here and throughout the description unless otherwise defined, refers to the relative weight of the respective component based on the overall weight of the formulation.

The term "extract" is to be understood as the liquid substance resulting of an extraction process of raw material such as plant material, preferably by use of a solvent. For the purpose of the current invention, the term "extract" equally applies to a component which is directly obtained from an extract, such as a spray dried extract.

The term "component of a composition" is to be understood as a constituent of the composition which is comprised of one or more active ingredients.

The term "excipient" is to be understood as a natural or synthetic substance formulated alongside the active ingredient of formulation included for the purpose of bulking-up formulations that contain active ingredients or to confer a therapeutic enhancement on the active ingredient in the final dosage form, such as facilitating drug absorption or solubility, or to aid in the manufacturing of the formulation.

The term "flowing agent" is to be understood as a substance which is added to a (solid) formulation to improve its flowability.

The term "anticaking agent" is to be understood as a substance which prevents the formation of lumps (caking).

The term "binding agent" is to be understood as a substance which holds ingredients together, ensuring that tablets and granules can be formed.

The term "bulking agent" is to be understood as a substance which is a diluting agent or for providing substance to a formulation. A bulking agent is generally inactive.

The term "herbal" is to be understood as a component which is derived of or consists or comprises herbs as main constituent. Herbal derivatives are to be understood as components which are mainly derived from herbs or herbal constituents, such as but not limiting to extracts, powders, dried herbal parts or tinctures.

The term "botanical (material) derived component" is to be understood as a component which is mainly derived from a plant or plant parts, said derivatives may be extracts, powders, dried plant parts or tinctures.

In a first aspect, the current invention relates to a composition for use in treating symptoms relating to premenstrual syndrome (PMS), said composition comprises at least one pollen or pistil extract or an extract of a combination of pollen and pistil as first active ingredient and at least one second active ingredient, said second active ingredients is a herbal or botanical derived component suitable of treating depressive and/or dysphoria symptoms linked to PMS.

The inventors of the current invention have surprisingly found that compositions comprised of an extract of pollen and optionally pistils, generally known in the art to be effective for alleviating or treating symptoms relating to PMS, are less efficient or in some patients even not useful when those PMS symptoms relate to depression, dysphoria and mood swings. As a result, a subpopulation of patients seeking for a treatment or alleviation of their PMS related symptoms will not or only inefficiently be helped when treated with compositions comprising pollen extract alone. This was hitherto not known. Although the reason behind the lack of activity of the pollen extract formulations is not known, it is deemed that the reason can be found in a biochemical difference between the patient groups that report dysphoria, depression and/or mood swings as one of the symptoms of PMS. Women with dysphoria and depression as main concern do not retain water prior to menses and do not suffer from weight gain, whereas women who suffer from a more classical symptomatic pattern did report water retention. Hence, it is deemed that a biological and/or biochemical cause lies at the basis of the observed differences in patient groups and effectiveness in treatment of the pollen extract based formulations.

As a consequence, the inventors of the current invention sought and found a method of provide to PMS patients a formulation that provides a solution for all PMS related symptoms, including those mainly observing a problem with dysphoria, depression and/or mood swings.

Said second active ingredient, suitable of treating depressive and/or dysphoria symptoms linked to PMS may be any active ingredient known in the art to be effective against depressive and/or dysphoria symptoms.
The second active ingredient is preferably a component which is derived from botanicals chosen from *Hypericum sp.,* such as *Hypericum perforatum* (St. John's wort), *Crocus sp.,* such as *Crocus sativus, Lavendula sp.* (lavender), *Rhodiola sp.* such as *Rhodiala rosea* (golden root), *Echium sp.* such as *Echium amoenum, Verbena sp.* such as *Verbena officialis* (vervain), *Avena sp.* such as *Verbena sativa* (green oats), *Eleutherococcus sp.* such as *Eleutherococcus senticosus, Leonurus sp.* such as *Leonurus cardia* (motherwort) and *Schisandra sp.* such as *Schisandra chinensis.*

Components derived from the latter are known to have an effect on treating patients which suffer from depressive and/or dysphoria symptoms. Although some of these botanicals have been reported to show a reduction in PMS symptoms as well when administered to patients, none of these are effective for the broad spectrum of symptoms relating to PMS.

However, by combination with the first active ingredient, a composition is provided which is able to treat or alleviate the majority if not all PMS related symptoms which are recognized today, including dysphoria and/or depression.

In another or further embodiment, said second active ingredient, suitable of treating depressive and/or dysphoria symptoms linked to PMS is safranal and/or (picro)crocin or a (botanical) source of safranal and/or (picro)crocin.

Safranal and (picro)crocin which are generally found in saffron, have been previously reported as being useful against treating or preventing depression and/or dysphoria, or symptoms relating to the latter disorders. The inventors of the current invention have found that by supplementing a composition comprising a pollen or pistil extract with safranal and/or (picro)crocin or a (botanical) source of the latter, the majority of women, if not all, suffering from PMS symptoms, including those with depression and dysphoria as main concern, can be treated or helped.

In a further or other embodiment, said active ingredient, suitable of treating depressive and/or dysphoria symptoms linked to PMS is saffron or a derivative of saffron, such as a saffron extract. Said source of saffron is preferably Crocus sativus. In a most preferred embodiment, said saffron extract is an extract of dried petal of Crocus sativus.

The usefulness of saffron or a saffron extract in treating or preventing symptoms relating to depression or dysphoria in PMS women has been described before (see Agha-Hosseini et al., 2008, In international journal of obstetrics and gynaecology).

By preference, the amount of safranal and/or (picro)crocin in the saffron extract will be at least 0.1%; in a further, more preferred embodiment, said amount will be between 0.1 and 10%.

In another embodiment, the amount of safranal and/or (picro)crocin in the final composition will be at least 0.001% by weight of the total composition, more preferably between 0.001% and 1%. This amount was found sufficient to provide an optimal effect.

The composition of the current invention further comprises a pollen and/or pistil extract, which is also known to act effectively against the vast majority of PMS related symptoms, except for depression and/or dysphoria. Said extract of pollen or pistil is by preference derived from plants belonging to the Graminae and/or Pinacea family.

More by preference, these extracts originate from plant material originating from *Zea mays L., Secale cereale L., Dactylis glomerata L., Pinus sylvestris L.* or a mixture thereof.

Preferably, the plant material used for the extracts is freshly harvested. The pollens used for the current invention can be pollen collected by insects (such as bee pollen) or collected by human intervention. Bee pollen for instance contains pollen, but also nectar and saliva from bees. Pollen collected via human intervention is devoid of such additional ingredients. By preference, said pollens of the current compositions are obtained by human intervention only.

Advantageously, the composition of the current invention comprises a component which is derived from a mixture of at least separate two extracts, whereby a first extract originates from corn pistil and from pollen selected from corn, rye, cocksfoot, pine or any combination thereof; and a second extract originating from corn pollen and pistil.

Preferably, said first extract originates from a mixture of at least corn pollen, rye pollen, cocksfoot pollen, pine pollen and corn pistil.

In another embodiment, said composition comprises a first and a second component, whereby said fist component is derived from an extract originating from pollen selected from corn, rye, pine or a combination thereof and a second component, whereby said second component is derived from an extract originating from corn pistil and pollen selected from corn, rye, cocksfoot, pine or any combination thereof.

The inventors found that the best results were obtained by making use of extract derived from a mixture of plants.

By preference, the concentration of first active ingredient, being the pollen and/or pistil extract, in the composition is the same or higher than the concentration of the second active ingredient, suitable of treating depressive and/or dysphoria symptoms linked to PMS.

By preference, said first and second ingredient will be present at a specific ratio. Preferably, the ratio between said first and second active ingredient in said composition is between 1:1 and 20:1, more preferably between 1:1 and 10:1.

The active ingredients of plant origin preferably comprise at least 40% by weight of the total composition, more preferably at least 40% by weight of the total composition, more preferably between 40 and 75% by weight of the total composition. The latter ensures optimal activity of the active ingredients in the composition.

In a preferred embodiment, said composition is free of phytoestrogens and/or does not exhibit any phytoestrogenic activity. Phytoestrogens are naturally-occurring plant compounds that are structurally and/or functionally similar to mammalian estrogens and their active metabolites. Due to their similarities with estrogens, phytoestrogens have been reported to act as antagonists of estrogens. Simultaneously to this antagonistic effect, they are also reported to show estrogenic activity. Although phytoestrogens have been linked to various beneficial effects in humans (e.g. for reducing the risk of coronary artery disease), phytoestrogens have also been reported to illicit negative reactions. For instance, some phytoestrogens have been reported to aggravate PMS symptoms. As a consequence, the composition of the current invention preferably does not exhibit any phytoestrogenic activity, in order to avoid any negative effect which could possibly be linked to the latter.

The composition may take any suitable form known in the art, such as a tablet, a capsule (hard or soft shell), a powder, a liquid such as an ampoule or syrup. In a most preferred embodiment, said composition is a tablet, a capsule, a soft-gel, semi-solid, solid, liquid or a powder.

The active ingredients may be provided in the form of granules, powder or crystalline form, as a component of the compositions according to the current invention. This may be achieved by freeze-drying, spray-drying, drum-drying or vacuum drying of the obtained extracts.

In a further preferred embodiment, the compositions according to the current invention may comprise excipients chosen from the group of additives such as flowing agents, binding agents, flavors, sweeteners, , and/or coating agents.

Solid compositions may also be coated using any pharmaceutically acceptable colored coating agents to improve their appearance and/or facilitate, patient identification of the product and unit dosage level.

In another or further embodiment, said composition comprises one or more vitamins selected from the group of vitamin A, vitamin B, vitamin C, vitamin D, vitamin E, vitamin K or any combination thereof. In a preferred embodiment, vitamin E is added to the composition. Vitamin E has been reported to have a beneficial effect on PMS symptoms and shows anti-oxidative activity.

The composition according to the current invention may be a pharmaceutical product, medical device, dietary or food supplement, health supplement, medical/dietetic food in an oral form.

In a preferred embodiment, a composition of the current invention will comprise:
- between 35 and 60 % by weight of one or more components derived of a pollen and/or pistil extract; more preferably at least 40%
- between 5 and 15% of a saffron extract or component derived from a saffron extract; more preferably at least 6%
- optionally, between 1 and 5% vitamin E; more preferably at least 3%
- excipients such as bulking, flowing and/or coating agents.

The invention is further described by the following non-limiting examples which further illustrate the invention, and are not intended to, nor should they be interpreted to, limit the scope of the invention.

### EXPERIMENTAL DATA AND EXAMPLES

### Compositions on the basis of pollen and optionally pistil extracts perform poorly when treating or preventing dysphoria and depression in women suffering from PMS

A clinical trial was performed including 120 patients who suffer from one or more symptoms relating to PMS, which was examined prior to the start of the study. The study was a multicenter out patient randomized, placebo controlled study approved by the Ethical Committee and conducted according to good clinical practice. Each group was subdivided, whereby one group was given a placebo twice a day and a second group a composition on the basis of an extract of pollen and pistil (Sérélys^{®} classic), equally twice a day.

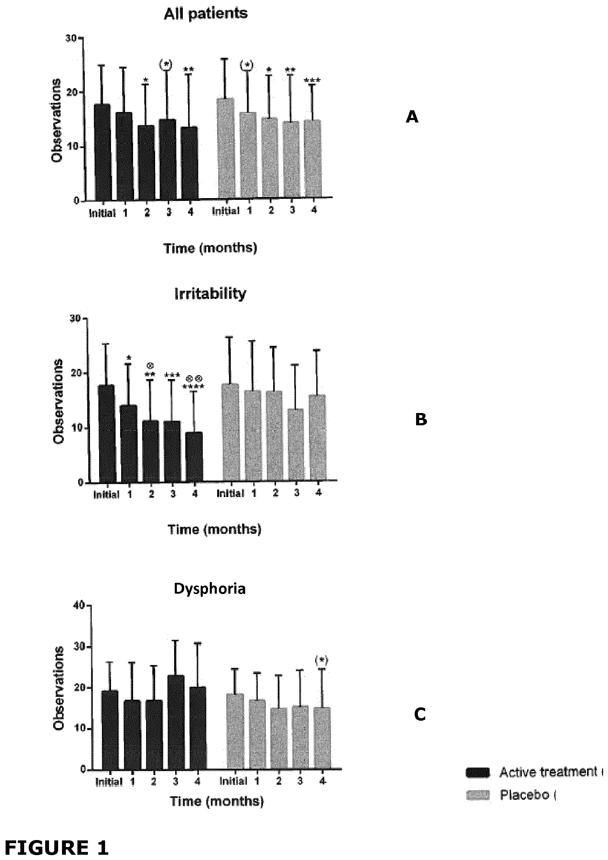

Observer evaluation of all patients enrolled in the trial, without a subdivision for symptoms, showed no significance difference between the group receiving the active treatment and the group receiving the placebo (figure 1, graph A). This was surprising and contrary to previous studies.

However, it was found that the population of patients could be further subdivided on the basis of their observed symptoms. Whereas one group of patients suffered from classical PMS symptoms such as irritability, anger, short fuse, etc.; a second group reported mainly dysphoria and/or depression as concern.

When looking into results of these subgroups, the group of patients reporting classic symptoms such as irritability showed significant changes between the group receiving the treatment and the placebo. This is especially evident after 2 and 4 months (see figure 1, graph B).

However, within the group reporting mainly dysphoria as most severe symptom, no effect could be observed (see figure 1, graph C, only a borderline alteration within the group was observed, which was not significant when comparing the two groups).

The observer data were further confirmed by the patients' own evaluation, which is summarized below in Table 1 and Table 2.

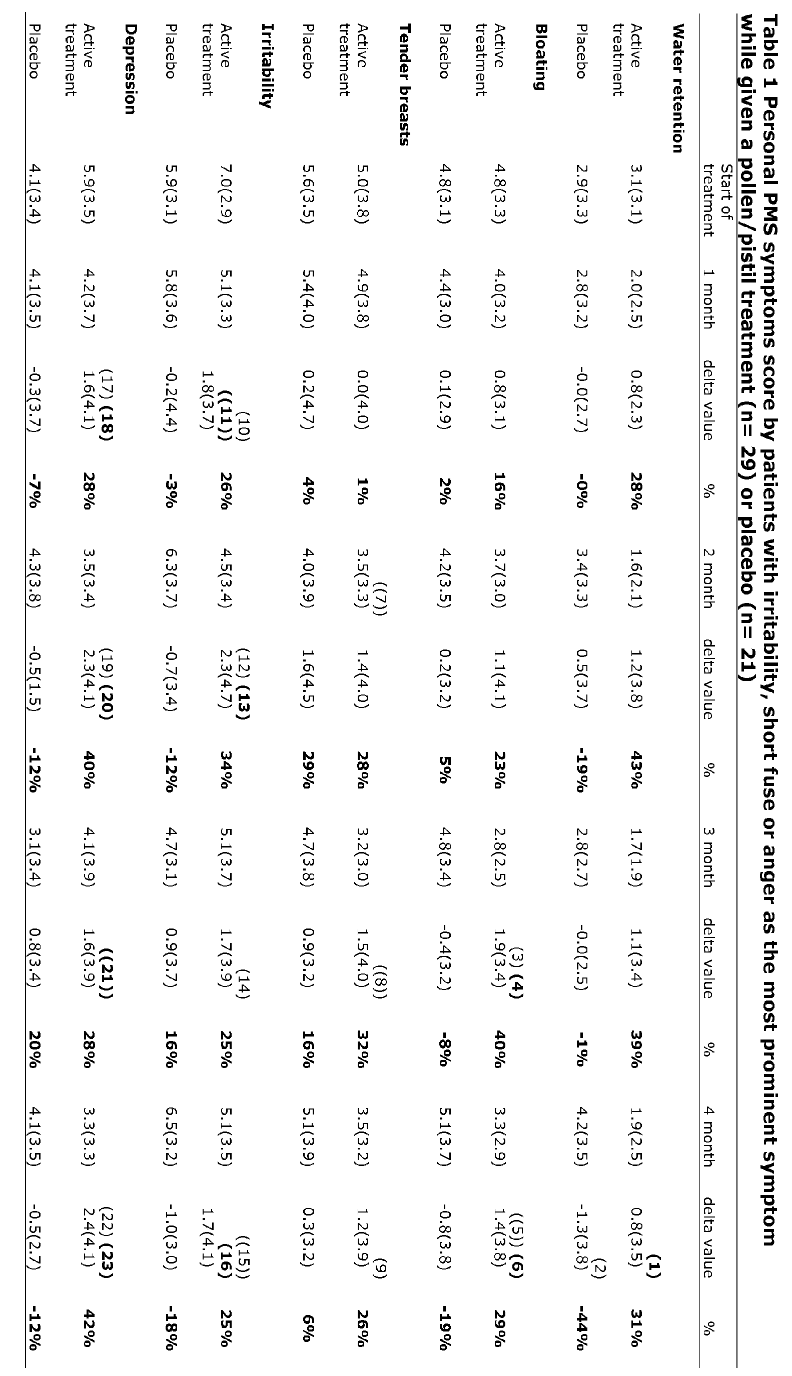

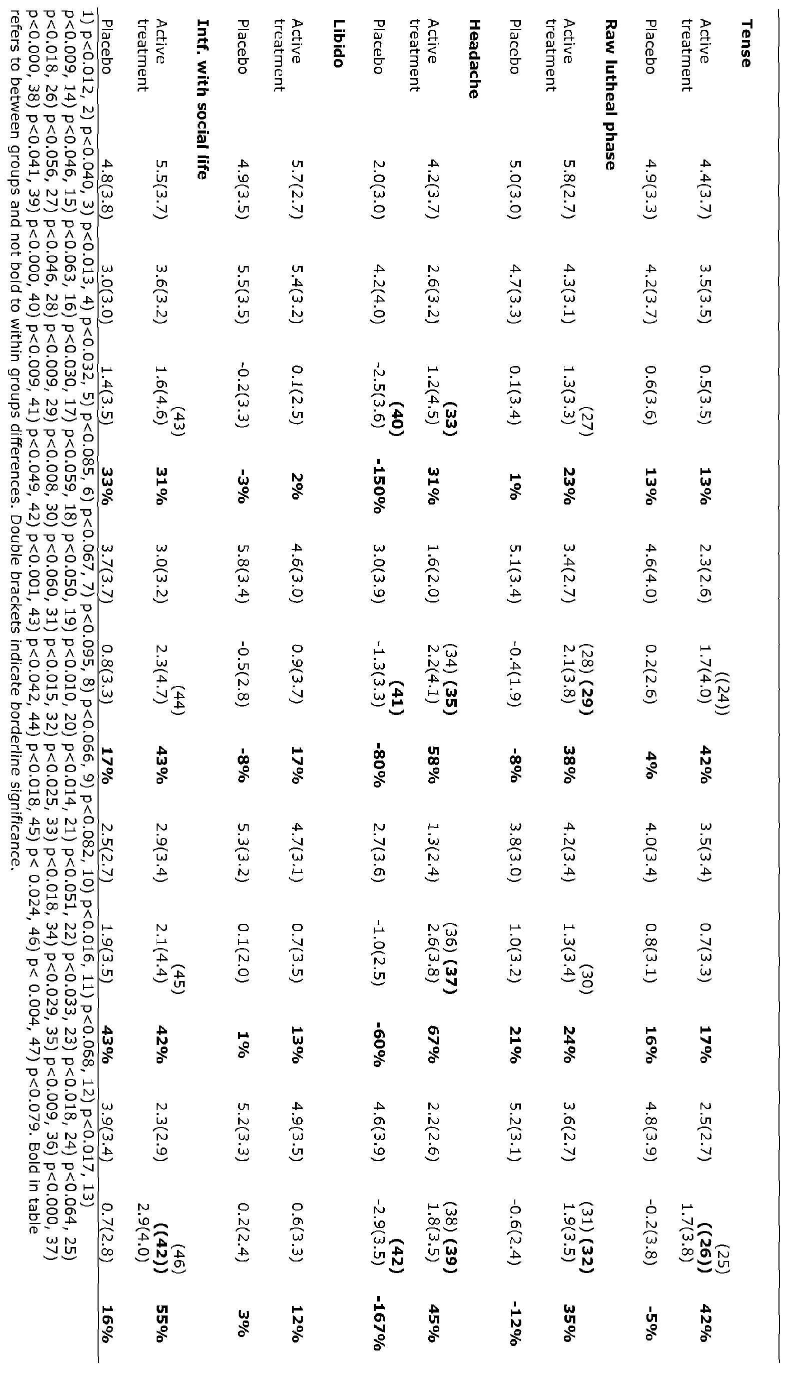

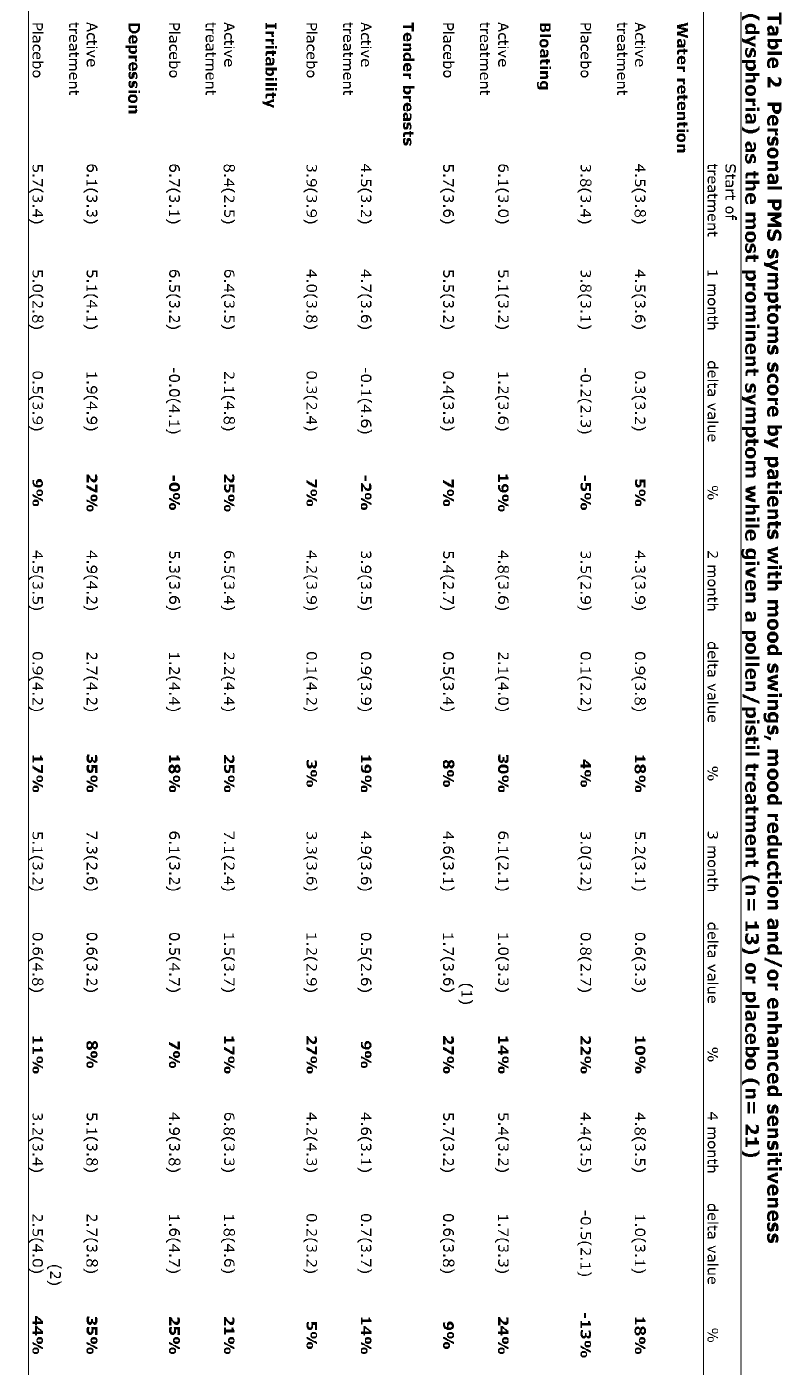

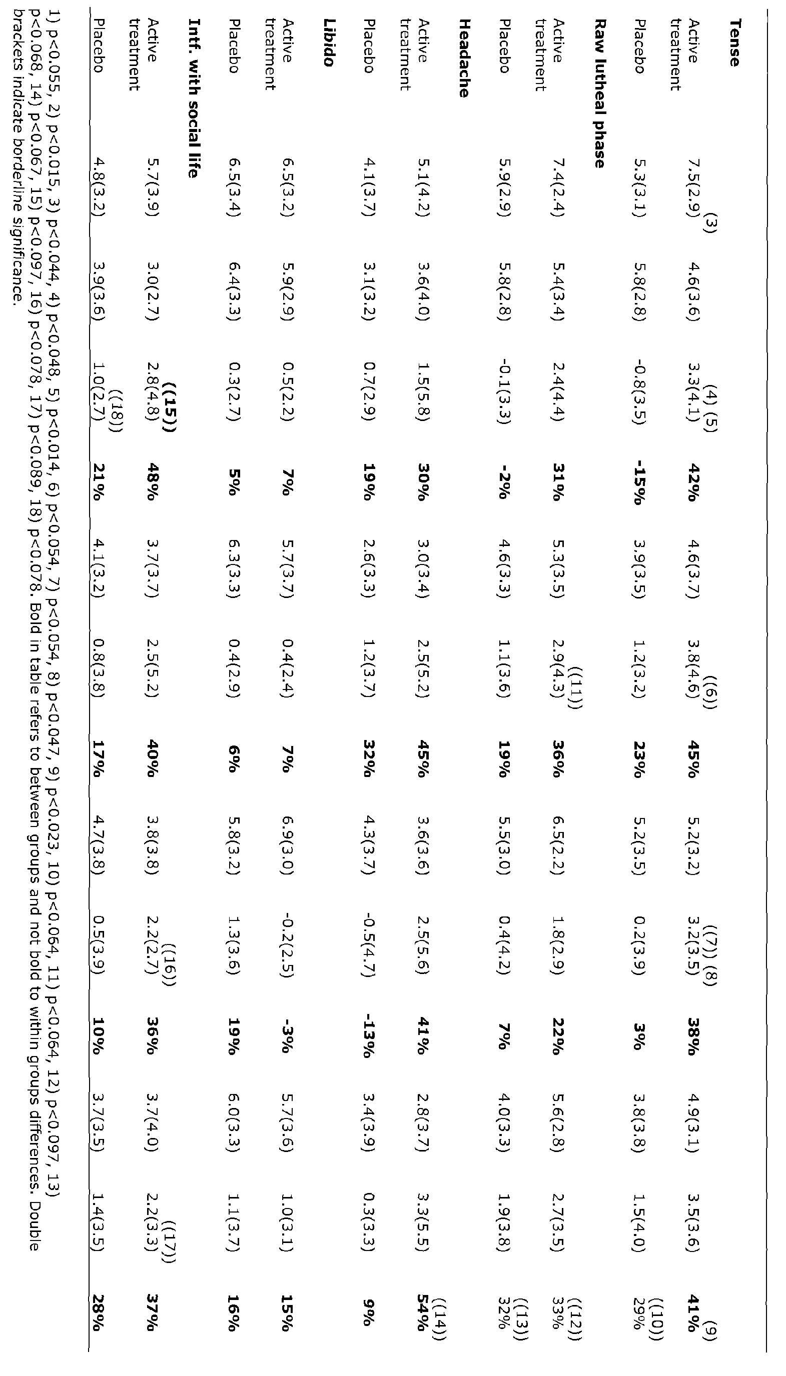

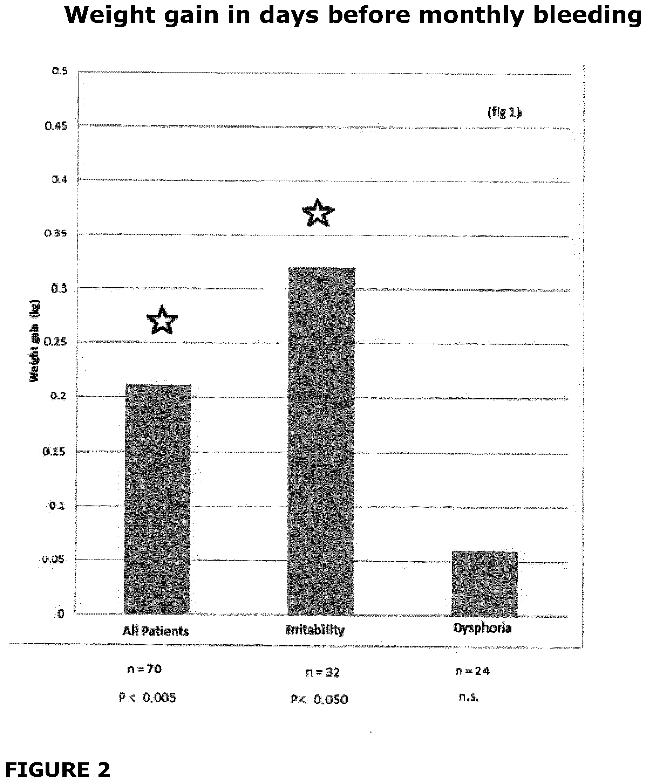

Hence, the results show that whereas a pollen/pistil composition indeed has a significant effect on women suffering from general PMS symptoms, such effect was not seen in the group reporting dysphoria and/or depression as main symptom. It was further found that this group did not report weight gain prior to the start of the menses (see figure 2), which indicates a biochemical/biological reason behind the observed differences.

### Use of a composition according to the current invention for treatment of Pre Menstrual Syndrome: two patient cases

A 37 year old woman who has not been pregnant for 4 years was suffering from severe PMS, characterized by tender breasts, depressive feelings and mood swings, especially seven days before menstruation. The symptoms were marked as severe. No further health problems were reported or detected. PAP smear was normal, as was the general gynecological examination. Also the blood test results were normal.

In a first instance, classical herbal treatment on the basis of pollen and pistil extract was given for 12 months. Whereas the patient did report an improvement in the tenderness of breasts, no results were observed with regard to the depressive feelings. No other medication was given.

The patient was subsequently given a composition according to the current invention, comprising the pollen and pistil extract, combined with saffron extract. The composition was given to the patient (once a day) for three months.

After three months, the patient was subjected to a questionnaire. From the latter, it showed that the patient observed a reduction in all symptoms, from a severe condition prior to taking the composition towards moderate or mild condition. No side effects were detected. It was decided that the patient should continue the treatment.

### Example for a composition according to the current invention

An example of a tablet according to the current invention is

| **Ingredients** | **Quantity mg per tablet (*without coating agent*)** |
|---|---|
| Pollen and pistil extract | 320.00 |
| Saffron extract (0.34% Safranal) | 50.0 |
| Vitamin E (anti-oxidant) | 22.2 |
| Microcrystalline cellulose (anticaking agent) | 232.4 |
| Magnesium Stearate (bulking agent) | 6.0 |
| Silicon dioxide (anticaking agent) | 6.0 |
| **TOTAL (without coating agent)** | **636.6** |

As coating agent, aquapolish is used. The amount of coating agent is at least 2.5% of the bulk tablet weight (approx. 15.9 mg), which makes the total weight of the tablet around 652.5 mg

## Claims

1. A composition for use in treating symptoms relating to premenstrual syndrome (PMS) **characterized in that** said composition comprises at least one pollen or pistil extract or a combination of the latter as first active ingredient and at least one second active ingredient, said second active ingredient is herbal or botanical material derived component and suitable of treating depressive and/or dysphoria symptoms linked to PMS.

2. Composition according to claim 1, **characterized in that** said second active ingredient is a component derived from botanicals chosen from *Hypericum sp.* such as St-John's wort, *Crocus sp.* such as Crocus sativus, *Lavendula sp.* such as lavender, *Rhodiola sp.* such as *Rhodiola rosea* (golden root), *Echium sp.* such as *Echium amoenum, Verbena sp.* such as *Verbena officialis* (vervain), *Avena sp.* such as *Avena sativa* (green oats), *Eleutherococcus sp.* such as *Eleutherococcus senticosus, Leonurus sp.* such as *Leonurus cardia* (motherwort) and *Schisandra sp.* such as *Schisandra chinensis.*

3. Composition for use according to claim 1, **characterized in that** said second active ingredient, suitable of treating depressive and/or dysphoria symptoms linked to PMS comprises safranal and/or (picro)crocin or a source of safranal and/or (picro)crocin.

4. Composition for use according to claim 3, **characterized in that** said source of safranal and/or (picro)crocin is saffron or a derivative of saffron.

5. Composition for use according to claim 4, **characterized in that** saffron or derivative thereof is derived from Crocus sativus.

6. Composition for use according to claim 4 or 5, **characterized in that** said derivative of saffron is a saffron extract.

7. Composition for use according to any of the previous claims, **characterized in that** said extract of pollen or pistil is derived from plants belonging to the Graminae and/or Pinacea family.

8. Composition for use according to claim 7, **characterized in that** said extract of pollen or pistil extract is derived from plants selected from the group of corn, rye, cocksfoot, pine or a combination thereof.

9. Composition for use according to any of the previous claims, **characterized in that** said composition is a pharmaceutical product, medical device, dietary or food supplement, health supplement, medical/dietic food in an oral form.

10. Composition for use according to any of the previous claims, **characterized in that** said composition is a tablet, a capsule, soft gel, semi-solid, solid, liquid or a powder.

11. Composition for use according to any of the previous claims, **characterized in that** said composition further comprises one or more vitamins selected from the group of vitamin A, vitamin B, vitamin C, vitamin D, vitamin E, vitamin K or any combination thereof.

12. Composition for use according to any of the previous claims, comprising excipients chosen from the group of flowing agents, binding agents, flavors, sweeteners and/or coating agents.
